# EUROPEAN PATENT APPLICATION

(11) **EP 4 574 045 A1**
(43) Date of publication of application: **25.06.2025**
(21) Application number: 24221994.7
(22) Date of filing: 20.12.2024
(51) Int. Cl.: A61B 5/367, A61B 5/00

(54) **METHOD AND SYSTEM FOR DISPLAYING INFORMATION DURING A CARDIAC OPERATION**

(30) Priority: 21.12.2023 US 202318392611
(71) Applicant: Biosense Webster (Israel) Ltd., 2066717 Yokneam (IL)
(72) Inventor: AVRY, Aviv Meir, 2066717 Yokneam (IL); SPITZER, Aviad, 2066717 Yokneam (IL); BUBAR, Zachary P., 2066717 Yokneam (IL)
(74) Representative: Carpmaels & Ransford LLP

(57) **Abstract**

A method, apparatus and computer program product, the method comprising: obtaining a plurality of electrical signals from a plurality of electrodes disposed on a catheter inserted into a chamber of a heart of a patient, wherein each electrical signal provides information about electrical activity in locations within the heart, and wherein each location is associated with a value for each attribute from a plurality of attributes; receiving from a user via a user interface a selection of a filtering criteria for the at least one location, the filtering criteria associated with at least one attribute from the plurality of attributes; applying the filtering criteria for identifying a subset of locations complying with the filtering criteria from the at least one location; updating a map of electrical activity within the heart, based only upon the subset of locations; and displaying the map of the electrical activity.

## Description

### FIELD OF THE DISCLOSURE

This disclosure relates to a method for selecting information to be displayed to a physician during a cardiac operation, using the information and for updating a view of the electrical activity within the heart.

### BACKGROUND OF THE DISCLOSURE

Arrhythmias may be caused by problems with the electrical conduction system of the heart, and in particular electrical activity in one or more points or areas on a wall of a heart chamber. Atrial fibrillation is an arrhythmia characterized by disorganized signals that make the atria (left and/or right atria) squeeze very fast and in an asynchronous cardiac rhythm.

In order to assess the status of the patient and decide on the treatment, such as applying one or more ablations, it may be required to assess the electrical activity at multiple locations of the heart wall. This activity may be obtained from a plurality of electrodes positioned on a distal tip of an intracardiac catheter inserted into one or more chambers of the heart. A plurality of the obtained signals may be displayed to a user, such as a physician performing the operation. The obtained signals may also be used for generating a map of the electrical activity within the heart.

### BRIEF DESCRIPTION OF THE DRAWINGS

The present disclosure will be more fully understood from the following detailed description of the examples thereof, taken together with the drawings, in which:
Fig. 1 is a schematic, pictorial illustration of a catheter-based electrophysiological (EP) mapping and ablation system;
Fig. 2 shows an exemplary user interface for defining or modifying attributes and presets, in accordance with some exemplary embodiments of the disclosure;
Fig. 3 shows a view of a display during a cardiac operation after a first preset is applied, in accordance with some exemplary embodiments of the disclosure;
Fig. 4 shows a view of a display during a cardiac operation after a second preset is applied, in accordance with some exemplary embodiments of the disclosure;
Fig. 5 is a flowchart of steps in a method for displaying information during a cardiac operation, in accordance with some exemplary embodiments of the disclosure; and
Fig. 6 is a schematic block diagram of a computing platform for displaying information during a cardiac operation, in accordance with some exemplary embodiments of the disclosure.

### DETAILED DESCRIPTION OF EXAMPLES

In the following description, numerous specific details are set forth in order to provide a thorough understanding of the present invention. It will be apparent to one skilled in the art, however, that the present invention may be practiced without these specific details. In other instances, well-known circuits, control logic, and the details of computer program instructions for conventional algorithms and processes have not been shown in detail in order not to obscure the present invention unnecessarily.

Software programming code, which embodies aspects of the present invention, is typically maintained in permanent storage, such as a computer readable medium. In a client-server environment, such software programming code may be stored on a client or a server. The software programming code may be embodied on any of a variety of known media for use with a data processing system. This includes, but is not limited to, magnetic and optical storage devices such as disk drives, magnetic tape, compact discs (CD's), digital video discs (DVD's), and computer instruction signals embodied in a transmission medium with or without a carrier wave upon which the signals are modulated. For example, the transmission medium may include a communications network, such as the Internet. In addition, while the invention may be embodied in computer software, the functions necessary to implement the invention may alternatively be embodied in part or in whole using hardware components such as application-specific integrated circuits or other hardware, or some combination of hardware components and software.

### OVERVIEW

Arrhythmogenic tissue related to atrial fibrillation may be identified by inspecting Intracardiac Electrogram (IEGM) at one or more locations over an atria wall, e.g., inner wall, to detect the local potential caused by depolarization in each of the one or more locations.

The IEGM is typically detected with one or more electrodes on a distal tip of an intracardiac catheter. In some example embodiments, the intracardiac catheter additionally includes a position sensor configured to track position of the distal tip.

Modern catheters have a plurality of electrodes distributed over a plurality of splines of the catheter, where at any given time, each electrode samples the electrical activity at a location. For example, some catheters may have dozens or even a hundred electrodes, and may thus collect information related to thousands of points within the heart, which amounts to a significant volume of data. The data may be used for generating a map showing an accurate geometric reconstruction of the heart, and an overall view of the electrical activity within the heart. The electrical activity may be represented graphically by color coding, pattern coding, or the like, of areas of the map according to the electrical activity at each location as measured by an electrode that has been present at the location.

However, this abundance of data also poses a challenge, as it may not enable the user, such as a physician or a clinical assistant (CAS), to concentrate on certain aspects of the operation or certain areas of the heart. For example, a user may be interested in seeing electrical activity information related only to areas of the heart that lie within scar border zones where arrythmia critical isthmuses exists. In another example, the user may be interested in seeing only areas where the late annotation time (LAT) has certain values, or the like.

In currently available systems and methods, a user may be presented with a list of the locations that have been sampled by an electrode and have been used in the construction of the heart map. The user may be further presented with the color-coded map based on all available locations and the electrical activity therein. The user may manually select individual locations to be included or excluded from being considered in generating the map and included in the view. However, due to the large number of electrodes in advanced catheters and the huge number of locations, this approach is hard to implement efficiently as it may take a long time for the physician to select the locations and identify within the map each location in the list. Such selection, beyond being infeasible, may also provide inaccurate results, as the physician may make mistakes in identifying the desired locations.

Thus, in accordance with some examples of the disclosure, the user may be presented with a user interface for selecting a subset of the available locations according to one or more attributes associated with each location. The user interface may comprise a list of attributes, and one or more possible values applicable to each such attribute. The user may then select the attributes to be checked and the respective required value or values for each attribute. The heart map may then be updated to indicate the electrical activity, based only on the locations that comply with the values of the applicable attributes. Thus, if the selection eliminates locations in a certain area, this area will not have an indication, such as a color indication, to the electrical activity.

It is appreciated, however, that the geometrical reconstruction of the heart map may be based on the locations of all available locations, such that the map structure and resolution is maintained, while fewer locations are indicated and contribute to the display of the electrical activity.

The reduction in the number of locations enables a physician to concentrate on the required aspects, on certain areas such as scar areas, or the like.

Some attributes may be binary, such as tissue proximity index (TPI) indicating whether the location is on the heart wall or not, such that the user may select only the locations that are on the heart wall, and eliminate the internal locations from the map and list. Other attributes, such as voltage or impedance, may be associated with a numeric range, such that the user may select only the locations having a value within a desired range (or belonging to a collection of selected values in case of a discrete attribute).

It is appreciated that for the non-selected attributes, all values are acceptable and no location is eliminated from the calculation and list due to its value for one of these attributes.

It is also appreciated that the user interface may enable opt-in such that the user may select which attributes to apply, or opt-out where the user may select which attributes not to apply.

One or more collections of attributes and corresponding values may be defined, stored and displayed as presets, such that the user can apply all attributes associated with a preset in a single action, without having to indicate each attribute and its corresponding values. The use may also apply a plurality of presets. Thus, one or more presets, whether defined globally as a default, or by the user, may be stored and displayed for the user to select from.

The values of attributes in presets may change according to the values of the sampled locations. for example, if for the attribute of voltage a preset indicates a value range of 0.5-1.5mV, if none of the sampled locations has a value exceeding 1.2mV, the preset may be updated for the current operation to display and enable the user to select a range of 0.5-1.2mV.

The system and method provide for advanced filtering of locations, according to one or more specific attributes. Additionally, a physician wishing to display only specific locations on a map, need not review all the available locations and decide which ones are to be used. Since each map can be based on tens of thousands of locations, selecting individual locations as in currently available systems is clearly infeasible. Thus, the disclosed method and system provide for automatically selecting locations based on selected attribute combinations. The selective and dynamic coloring of the heart map provides for improved user experience and streamlining the work, thereby also reducing the operation time.

Additionally, the definition, storage and customizable definition of criteria and presets provide for improved efficiency as well flexibility of the system, since a user may not need to define the presets anew every time, but may be able to change it if required.

### SYSTEM DESCRIPTION

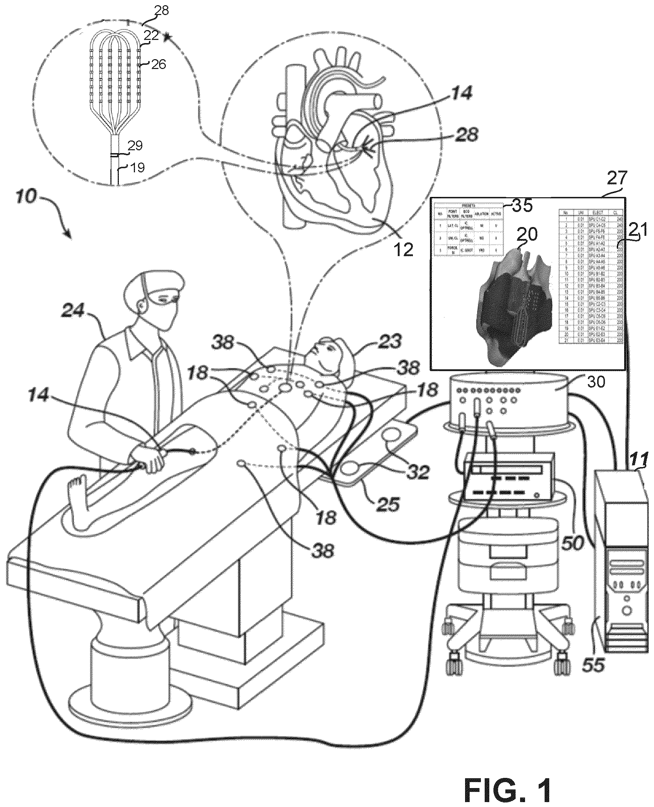

Reference is made to Fig. 1 showing an example catheter-based electrophysiology mapping and ablation system 10. System 10 may include multiple catheters, which may be percutaneously inserted by a physician 24 through the vascular system of a patient 23 into a chamber or vascular structure of a heart 12. Typically, a delivery sheath catheter is inserted into the left or right atrium near a desired location in heart 12. Thereafter, one or more catheters may be inserted into the delivery sheath catheter so as to arrive at the desired location in heart 12. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 may place electrode assembly 28 of catheter 14 in contact with the heart wall for sensing a target site in heart 12. For ablation, physician 24 may similarly place a distal end of an ablation catheter in contact with a target site for ablating tissue.

Catheter 14 is an exemplary catheter that is basket-shaped and includes one and preferably multiple electrodes 26 optionally distributed over a plurality of splines 22 at electrode assembly 28 and configured to sense the IEGM signals. Each electrode 26 is connected via a wire (not shown) going through or attached to spline 22 towards Patient interface unit (PIU) 30 detailed below. Splines 22 may be coupled to shaft 19 of electrode assembly 28. However, it is appreciated that the disclosure is not limited to a basket-shaped electrode assembly 28, but is applicable to any type of catheter having any number of splines and electrodes.

Catheter 14 may additionally include a position sensor 29 embedded in or near electrode assembly 28 for tracking position and orientation of electrode assembly 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. The real time position of electrode assembly 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6, 892, 091.

Additionally or alternatively, system 10 may include one or more electrode patches 38 positioned for skin contact on patient 23 to establish location reference for location pad 25 as well as impedance-based tracking of electrodes 26. For impedance-based tracking, electrical current is directed to electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

System 10 displays over display device 27 a preset selection pane 35, an anatomical map 20 of the heart showing the electrical activity within the hear using color coding, and a pane 21 of the attributes of a plurality of locations captured by electrodes 26 of catheter 14, the plurality of locations used for color coding the map.

Recorder 11 may include pacing capability for pacing the heart rhythm and/or may be electrically connected to a standalone pacer.

PIU 30 may be configured to establish electrical communication between catheters, other electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the electrodes 26 and for performing ECG calculations.

Workstation 55 includes memory, processor unit with memory or storage with appropriate operating software stored therein, and user interface capability. Workstation 55 may provide multiple functions, optionally including (1) modeling the endocardial anatomy in three-dimensions (3D) based upon the signals received from electrodes 26 and rendering the model or anatomical map 20 for display; (2) selecting, in accordance with the active presets, a subset of the locations for which an electrode 26 provided information; and (3) displaying on display device 27 the rendered anatomical map 20 as color- or pattern-coded in accordance with the selected locations, the presets and the selected location list. One commercial product embodying elements of system 10 is available as the CARTO^{™} 3 System, available from Biosense Webster, Inc., 31A Technology Drive, Irvine, CA 92618.

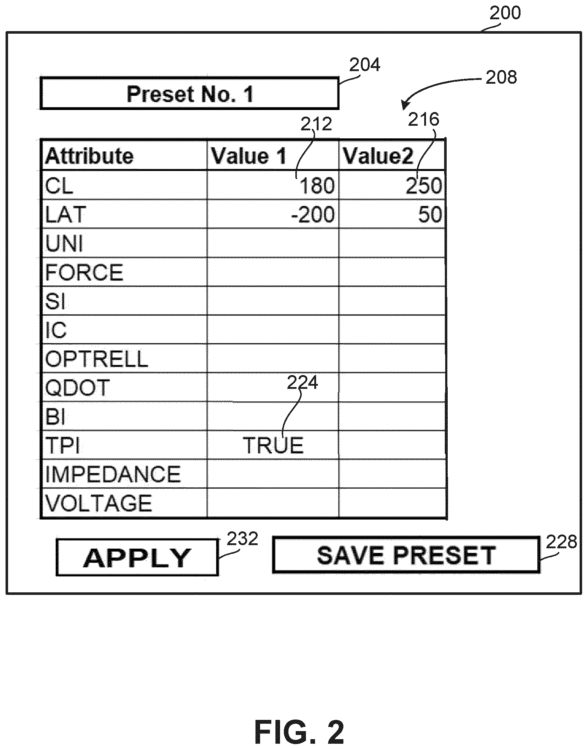

Reference is made to Fig. 2, showing an exemplary user interface for defining or modifying attributes and presets, in accordance with some exemplary embodiments of the disclosure.

The user interface, generally referenced 200, comprises a number or another identifier 204 for a preset being defined or modified.

User interface 200 further comprises table 208 for defining the attributes to be applied and their respective values. One or more values may be defined for each such attribute, indicating for example the range, allowed values, or a binary value. The values may be provided by a user typing text into a text box, using a slider, selecting from a list, or the like. In some embodiments, a set of one or more values may be selected, for example for an attribute having discrete values.

For example, for the attribute CL (cycle length), a first value (212) of 180 may be a minimal value, and a second value (216) of 250 may be a maximal value.

For binary attributes, such as TPI (tissue proximity), only one value 224 may be selected among the possible values, such as True/False.

Once the attributes and values are defined, a user may save the collection of attributes as a preset by pressing "Save Preset" button 228. In some embodiments, the user may be prompted to provide an indicative name for the preset.

In some embodiments, the user may apply ad-hoc the collection of active attributes to the currently available locations by pressing Apply button 232, without saving a preset.

It is appreciated that the values to be indicated, for example as first value 212 and second value 216, or as first value 224, may have default values suggested to a user. In some embodiments, the values a user may enter or select may be limited to the values available in the collection of locations, such that during an operation a user cannot select a value that is lower than a minimal value present in any of the points, a value that is higher than a maximal value present in any of the points, a binary value that is unavailable in any of the points, or the like.

An exemplary list of attributes for which values may be limited includes but is not limited to any one or more of the following:
Impedance, for which a lower and/or upper limit may be provided, and which may provide an indication for a scar tissue;
Voltage, for which a lower and/or upper limit may be provided;
LAT (late activation time), for which a lower and/or upper limit may be provided;
LAM (Late Annotation Mapping);
BI (a mean value obtained from bi-polar electrodes), for which a lower and/or upper limit may be provided;
TPI (tissue proximity), for which a True/False value may be provided, indicating whether the location is on the heart wall or internal;
CL (cycle length), for which a lower and/or upper limit may be provided;
one or more stability parameters of the signal;
fractionated signals, for which a True/False value may be provided; and
connected signals, for which a true/false value may be provided.

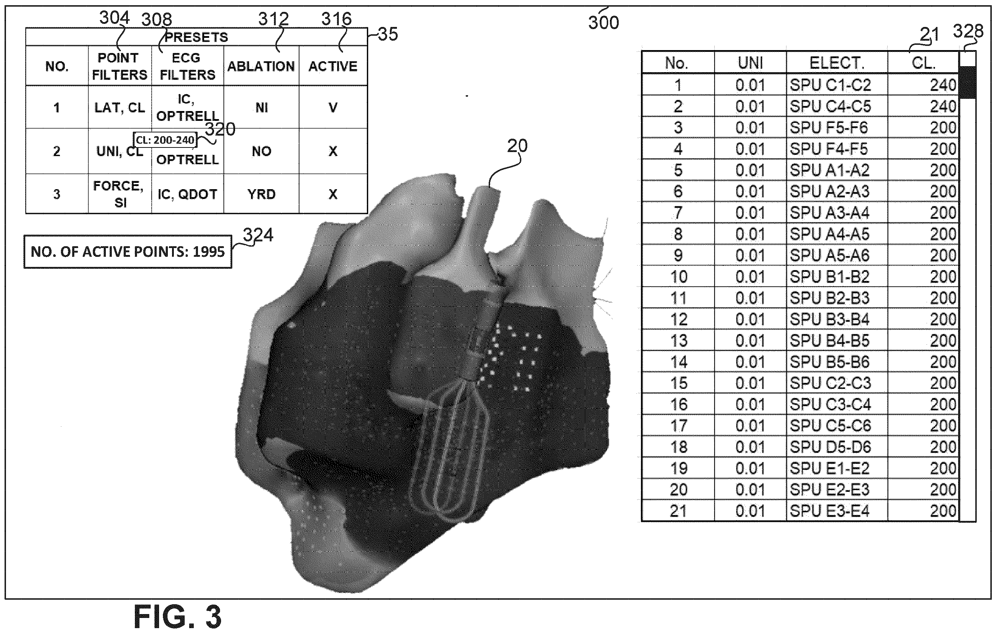

Referring now to Fig. 3, showing a view of a display during a cardiac operation after a first preset is applied, in accordance with some exemplary embodiments of the disclosure.

The view, generally referenced 300, is substantially identical to the view displayed on display 27 of Fig. 1.

View 300 shows preset pane 35, where the user can see three presets, numbered 1, 2, and 3. For each preset, the attributes associated with the preset are shown split to groups, such as point filters 304, ECG filters 308, and ablation filters 312. For each preset, the user may select, using the corresponding entry in "active" column 316 whether it is to be activated or not. In the example of Fig. 3, only preset no. 1 is activated.

It is appreciated that multiple presets may be activated simultaneously, such that the selected locations are those whose attributes comply with all active presets. For example, if one active preset limits the CL attribute to 170-240, and another active preset limits the CL attribute to 200-270, the actual used values will be 200-240.

The range may be further reduced according to the attribute values of the available locations. For example, if the minimal available value is 210, the range will be reduced to 210-240.

In some embodiments, if the user points at, or hovers over a certain attribute, such as CL, a popup window 320 may be displayed, indicating the values associated with this attribute in the current situation, such as the 210-240 range above. In other embodiments, the text may display the values as determined by the active presets, such as 200-240 above.

View 300 may further display, for example in text box 324, the number of active locations, i.e., the number of locations used for estimating the electrical activity in the heart and coloring the map under the current presets.

View 300 may further comprise a two-dimensional (2D) or three-dimensional (3D) map 20 of the heart, wherein map 20 can be manipulated by the user, e.g. rotated, scaled, zoomed, or the like.

View 300 may further comprise a list 21 of the locations that comply with all active presets, and thus used for estimating the electrical activity in the heart, and some of the attributes of these locations. In some embodiments, the displayed attributes may be attributes whose values are limited by the active preset(s).

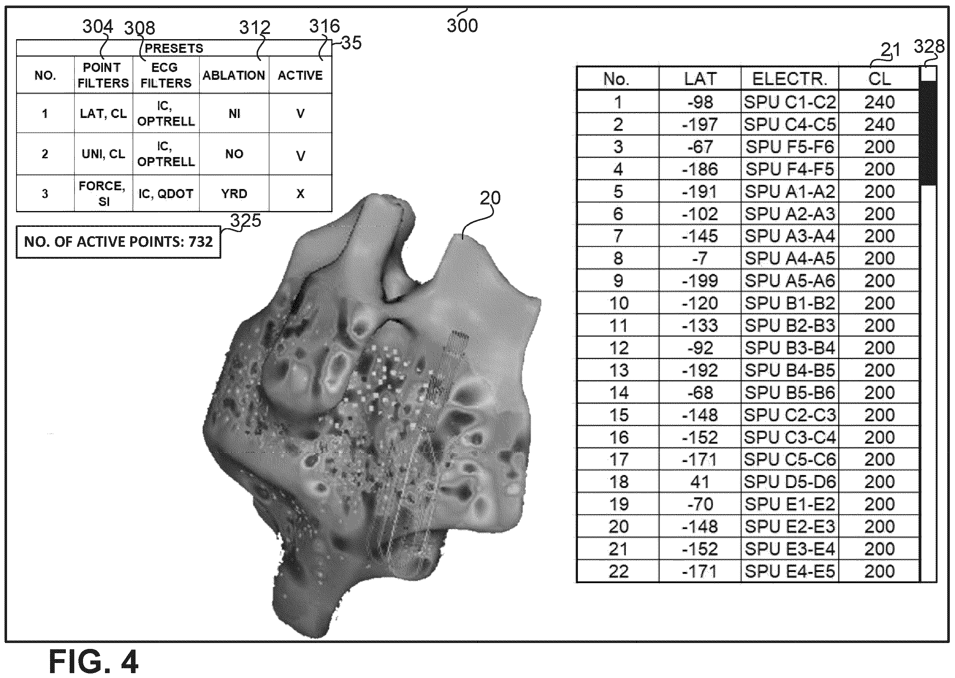

Referring now to Fig. 4, showing a view of a display during a cardiac operation after and presets no. 1 and no. 2 are activated. This preset selection reduces the number of active locations as shown in text box 325, from 1995 to 732. The reduction can also be observed by the size of scrollbar 328 relative to its size in Fig. 3.

It is seen that the color coding of map 20 in Fig. 4 is different from that of Fig. 3, as it is based on a different set of locations. However, the anatomical reconstruction of the heart is unchanged, as it is based on the location information obtained from the electrodes for all the points.

It is appreciated that multiple maps may be displayed, for example in different panes or different tabs of the display, such that the user can toggle among the different maps.

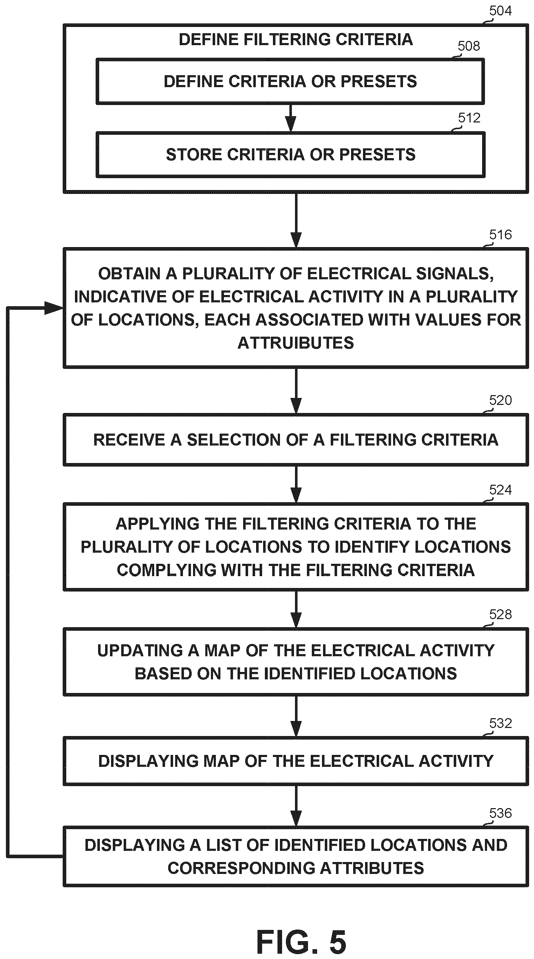

Referring now to Fig. 5, showing a flowchart of steps in a method for displaying information during a cardiac operation, in accordance with some exemplary embodiments of the disclosure.

On step 504, which may be a preliminary step, one or more filtering criteria may be defined. For example, definition step 504 may be performed upon configuration of a system by a manufacturer, during deployment of the system by a professional, or during or prior to using the system, by a user such as a physician.

Step 504 may include step 508 of defining one or more criteria related to an attribute of locations within a heart of a patient, or to a preset related to one or more such attributes. Each attribute comprised in the criteria or preset may be associated with one or more values, such as a numerical range(s), a binary value, a selection of discrete values, or the like.

On step 512, the criteria or preset may be stored at a storage device accessible to a computing platform associated with the operation, such as workstation 55.

On step 516, during a cardiac operation, a plurality of electrical signals may be obtained from electrodes of a catheter, wherein the electrical signals are indicative of electrical activity in a plurality of locations within or on the wall of the heart. It is appreciated that each signal may provide information related to a plurality of locations, as the electrodes are moved by the user. Each such location may be associated with a value for each of a plurality of attributes. The locations and the electrical signals may be useful in constructing a map of the heart, and coding of the map according to the electrical activity in each area of the heart.

On step 520, a selection of one or more filtering criteria, whether or not the criteria is a part of a preset or not, may be received. The filtering criteria may be selected from the predefined stored criteria or presets, or defined ad-hoc by a user of the system.

On step 524, the filtering criteria or preset may be applied towards the plurality of locations, to identify locations that comply with the filtering criteria or the preset. It is appreciated that a plurality of criteria or presets may be applied, such that only locations complying with all applied criteria or presets are identified.

On step 528, a map showing the electrical activity within the heart may be updated to indicate the electrical activity based only on the locations that complied with the criteria or presets. Thus, the coding of the map may be based on fewer locations than prior to applying the criteria or preset, but the updated map may provide an indication more suitable to the needs of the user.

On step 532, the updated map may be displayed to the user.

On step 536 the list of locations that complied with the criteria or presets may be displayed, while avoiding the display of the non-complying locations. In some embodiments, the values associated with one or more attributes for each location may be displayed as well.

The process may be repeated during the cardiac operation, and the criteria or preset may be re-applied on each iteration, for the newly received locations. It is appreciated that the selection of criteria or preset does not need to be repeated for each iteration, but only when there is a change in the user's selection.

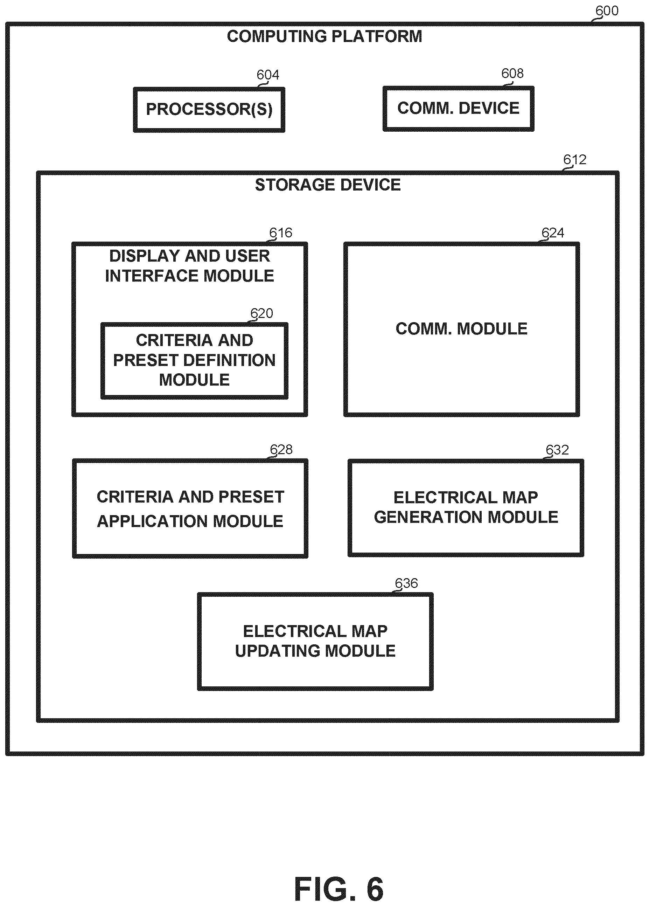

Referring now to Fig. 6, showing a block diagram of a computing platform 600 for displaying information during a cardiac operation, in accordance with some exemplary embodiments of the disclosure.

It is appreciated that computing platform 600 may be embedded within workstation 55, but may also be a standalone computing platform or embedded elsewhere and be in operative communication with workstation 55.

Computing platform 600 may be implemented as one or more computing platforms which may be operatively connected to each other. For example, one or more remote computing platforms, which may be implemented for example on a cloud computer. Other computing platforms may be a part of a computer network of the associated organization. In other embodiments, all the functionality may be provided by one or more computing platforms all being a part of the organization network.

Computing platform 600 may comprise one or more processors 604 located on the same computing platform or not, which may be one or more Central Processing Units (CPU), microprocessors, electronic circuits, Integrated Circuits (IC) or the like. Processor 604 may be configured to provide the required functionality, for example by loading to memory and activating the software modules stored on storage device 612 detailed below.

Computing platform 600 may comprise a communication device 608 for communicating with other devices or other computing platforms as necessary, for example obtaining information from a catheterization controller indicating locations and electrical measurements, storing data such as presets on remote storage devices, or the like. Communication module 608 may be adapted to interface with any communication channel such as Local Area Network (LAN), Wide Area Network (WAN), cellular network or the like, and use any relevant communication protocol.

Computing platform 600 may comprise a storage device 612, such as a hard disk drive, a Flash disk, a Random Access Memory (RAM), a memory chip, or the like. In some exemplary embodiments, storage device 612 may retain program code operative to cause processor 604 to perform acts associated with any of the modules listed below, or steps of the method of Fig. 5 above. The program code may comprise one or more executable units, such as functions, libraries, standalone programs or the like, adapted to execute instructions as detailed below.

Alternatively or additionally, the provided instructions may be stored on non-transitory tangible computer-readable media, such as magnetic, optical, or electronic memory.

Storage device 612 may comprise display and user interface module 616, for rendering a display to the user, to be displayed over display device 27, such as a map of the heart, location list, or the like. Display and user interface module 616 may also be operative in receiving instructions and operation parameters from the user, for example selecting criteria or presets. Display and user interface module 616 may comprise criteria and preset definition module 620, for defining one or more criteria or presets, including selecting attributes and corresponding values, as shown for example on Fig. 2 above.

Storage device 612 may comprise communication module 624 for transmitting and receiving data to and from other parts of the system or other systems through communication device 608, such as catheter locations, associated electrograms, user preferences, histograms, or the like.

Storage device 612 may comprise criteria and preset application module 628, for applying the selected criteria to the locations for which information was received from the electrodes, to identify the locations that comply with the criteria or presets. If a plurality of presets or criteria are selected which have a common attribute, the identified locations are those complying with the intersection of the of the defined values.

Storage device 612 may comprise electrical map generation module 632 for generating a geometric reconstruction of the heart and an electrical activity map indicated thereon, based on all available points.

Storage device 612 may comprise electrical map updating module 636 for updating an electrical activity map of the heart, to reflect electrical information associated only with the locations complying with the applied criteria or presets.

It is appreciated that the steps and modules disclosed above are in addition to the software, hardware, firmware or other modules required for operating the catheter, displaying the catheterization process, performing other calculations such as signal analysis and in particular complex fractionated electrogram (CFE) analysis, generating the heart map, or the like. Further details for methods and systems may be found, for example in US8676305, US9629567, incorporated herein by reference in their entirety for any purpose.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture instructions, machine instructions, machine dependent instructions, microcode, firmware instructions, state-setting data, or either source code or object code written in any combination of one or more programming languages, programming languages such as Java, C, C++, Python, or others. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

### EXAMPLES

### Example 1

A method comprising: obtaining a plurality of electrical signals from a plurality of electrodes disposed on a catheter inserted into one or more chambers of a heart of a patient, wherein each electrical signal from the plurality of electrical signals provides information about electrical activity in at least one location within the heart, and wherein each location of the at least one location is associated with a value for each attribute from a plurality of attributes; receiving from a user via a user interface a selection of a filtering criteria for the at least one location, the filtering criteria associated with at least one attribute from the plurality of attributes; applying the filtering criteria for identifying a subset of locations complying with the filtering criteria from the at least one location; updating a map of electrical activity within the heart, based only upon the subset of locations; and displaying the map of the electrical activity.

### Example 2

The method according to example 1, wherein performing said obtaining, said applying, said updating and said displaying, are performed repetitively.

### Example 3

The method according to example 1 or 2, further comprising displaying to a user a representation of information related to the subset of locations, while avoiding displaying information for other locations not complying with the filtering criteria.

### Example 4

The method according to any of the preceding examples, wherein the plurality of electrical signals are Electrocardiography (ECG) signals.

### Example 5

The method according to any of the preceding examples, wherein the at least one attribute is selected from the group consisting of: impedance; voltage; late annotation time (LAT) value at a location associated with the electrical signal; Late Annotation Mapping (LAM); Tissue Proximity Index (TPI); value obtained from bi-polar measurement (BI); cycle length (CL) of the electrical signal; at least one stability parameter of the signal; fractionated or non-fractionated signals; and connected or disconnected signals.

### Example 6

The method according to any of the preceding examples, wherein the at least one filtering criteria is comprised in at least one preset, and wherein applying the filtering criteria is performed by applying the at least one preset for identifying the subset of locations.

### Example 7

The method according to any of the preceding examples, further comprising defining the at least one filtering criteria.

### Example 8

The method according to example 7, wherein defining the at least one filtering criteria comprises defining at least one preset associated with the at least one attribute.

### Example 9

The method according to example 8, further comprising storing the at least one preset.

### Example 10

The method according to example 8, wherein defining the at least one filtering criteria comprises selecting the at least one attribute and at least one corresponding value.

### Example 11

The method according to example 10, wherein at least one corresponding value includes a minimal or a maximal value for a numerical range for the at least one attribute.

### Example 12

The method according to example 10, wherein the at least one corresponding value includes a binary value for the at least one attribute.

### Example 13

The method according to example 10, wherein the at least one corresponding value includes one or more discrete values for the at least one attribute.

### Example 14

The method according to example 8, wherein the user interface enables a user to select one or more presets to be applied, from the at least one preset.

### Example 15

The method according to example 14, wherein if a same attribute is comprised in at least two activated presets, the at least one corresponding value of the same attribute complies with limitations of the two presets.

### Example 16

The method according to example 14, wherein the at least one corresponding value of the attribute in a preset complies with available values of the at least one attribute within the at least one location.

### Example 17

A computerized apparatus having a processor coupled with a memory unit, the processor being adapted to perform the steps of: obtaining a plurality of electrical signals from a plurality of electrodes disposed on a catheter inserted into one or more chambers of a heart of a patient, wherein each electrical signal from the plurality of electrical signals provides information about electrical activity in at least one location within the heart, and wherein each location of the at least one location is associated with a value for each attribute from a plurality of attributes; receiving from a user via a user interface a selection of a filtering criteria for the at least one location, the filtering criteria associated with at least one attribute from the plurality of attributes; applying the filtering criteria for identifying a subset of locations complying with the filtering criteria from the at least one location; updating a map of electrical activity within the heart, based only upon the subset of locations; and displaying the map of the electrical activity.

### Example 18

The apparatus according to example 17, wherein the at least one filtering criteria is comprised in at least one preset, and wherein applying the filtering criteria is performed by applying the at least one preset for identifying the subset of locations.

### Example 19

The apparatus according to example 18, wherein the processor is further adapted to obtain a definition of the at least one preset, the at least one preset associated with the at least one attribute.

### Example 20

A computer program product comprising a non-transitory computer readable medium retaining program instructions, which instructions when read by a processor, cause the processor to perform: obtaining a plurality of electrical signals from a plurality of electrodes disposed on a catheter inserted into one or more chambers of a heart of a patient, wherein each electrical signal from the plurality of electrical signals provides information about electrical activity in at least one location within the heart, and wherein each location of the at least one location is associated with a value for each attribute from a plurality of attributes; receiving from a user via a user interface a selection of a filtering criteria for the at least one location, the filtering criteria associated with at least one attribute from the plurality of attributes; applying the filtering criteria for identifying a subset of locations complying with the filtering criteria from the at least one location; updating a map of electrical activity within the heart, based only upon the subset of locations; and displaying the map of the electrical activity.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method comprising:
obtaining a plurality of electrical signals from a plurality of electrodes disposed on a catheter inserted into one or more chambers of a heart of a patient, wherein each electrical signal from the plurality of electrical signals provides information about electrical activity in at least one location within the heart, and wherein each location of the at least one location is associated with a value for each attribute from a plurality of attributes;
receiving from a user via a user interface a selection of a filtering criteria for the at least one location, the filtering criteria associated with at least one attribute from the plurality of attributes;
applying the filtering criteria for identifying a subset of locations complying with the filtering criteria from the at least one location;
updating a map of electrical activity within the heart, based only upon the subset of locations; and
displaying the map of the electrical activity.

2. The method of Claim 1, wherein performing said obtaining, said applying, said updating and said displaying, are performed repetitively.

3. The method of Claim 1 or Claim 2, further comprising displaying to a user a representation of information related to the subset of locations, while avoiding displaying information for other locations not complying with the filtering criteria.

4. The method of any preceding Claim, wherein the plurality of electrical signals are Electrocardiography (ECG) signals.

5. The method of any preceding Claim, wherein the at least one attribute is selected from the group consisting of: impedance; voltage; late annotation time (LAT) value at a location associated with the electrical signal; Late Annotation Mapping (LAM); Tissue Proximity Index (TPI); value obtained from bi-polar measurement (BI); cycle length (CL) of the electrical signal; at least one stability parameter of the signal; fractionated or non-fractionated signals; and connected or disconnected signals.

6. The method of any preceding Claim, wherein the at least one filtering criteria is comprised in at least one preset, and wherein applying the filtering criteria is performed by applying the at least one preset for identifying the subset of locations.

7. The method of Claim 1, further comprising defining the at least one filtering criteria.

8. The method of Claim 7, wherein defining the at least one filtering criteria comprises defining at least one preset associated with the at least one attribute.

9. The method of Claim 8, further comprising storing the at least one preset.

10. The method of Claim 8, wherein defining the at least one filtering criteria comprises selecting the at least one attribute and at least one corresponding value.

11. The method of Claim 10, wherein at least one corresponding value includes a minimal or a maximal value for a numerical range for the at least one attribute, the at least one corresponding value includes a binary value for the at least one attribute, or the at least one corresponding value includes one or more discrete values for the at least one attribute.

12. The method of Claim 8, wherein the user interface enables a user to select one or more presets to be applied, from the at least one preset.

13. The method of Claim 12, wherein i) if a same attribute is comprised in at least two activated presets, the at least one corresponding value of the same attribute complies with limitations of the two presets, or ii)the at least one corresponding value of the attribute in a preset complies with available values of the at least one attribute within the at least one location.

14. A computerized apparatus having a processor coupled with a memory unit, the processor being adapted to perform the steps of:
obtaining a plurality of electrical signals from a plurality of electrodes disposed on a catheter inserted into one or more chambers of a heart of a patient, wherein each electrical signal from the plurality of electrical signals provides information about electrical activity in at least one location within the heart, and wherein each location of the at least one location is associated with a value for each attribute from a plurality of attributes;
receiving from a user via a user interface a selection of a filtering criteria for the at least one location, the filtering criteria associated with at least one attribute from the plurality of attributes;
applying the filtering criteria for identifying a subset of locations complying with the filtering criteria from the at least one location;
updating a map of electrical activity within the heart, based only upon the subset of locations; and
displaying the map of the electrical activity.

15. The apparatus of Claim 14, wherein the at least one filtering criteria is comprised in at least one preset, and wherein applying the filtering criteria is performed by applying the at least one preset for identifying the subset of locations, optionally wherein the processor is further adapted to obtain a definition of the at least one preset, the at least one preset associated with the at least one attribute.

16. A computer program product comprising a non-transitory computer readable storage medium retaining program instructions configured to cause a processor to perform actions, which program instructions implement:
obtaining a plurality of electrical signals from a plurality of electrodes disposed on a catheter inserted into one or more chambers of a heart of a patient, wherein each electrical signal from the plurality of electrical signals provides information about electrical activity in at least one location within the heart, and wherein each location of the at least one location is associated with a value for each attribute from a plurality of attributes;
receiving from a user via a user interface a selection of a filtering criteria for the at least one location, the filtering criteria associated with at least one attribute from the plurality of attributes;
applying the filtering criteria for identifying a subset of locations complying with the filtering criteria from the at least one location;
updating a map of electrical activity within the heart, based only upon the subset of locations; and
displaying the map of the electrical activity.
